# EUROPEAN PATENT SPECIFICATION

(11) **EP 0 461 662 B1**
(45) Date of publication and mention of the grant of the patent: **11.09.1996**
(21) Application number: 91109743.4
(22) Date of filing: 13.06.1991
(51) Int. Cl.: A61K 9/127

(54) **Process for preparing liposomes**
Verfahren zur Herstellung von Liposomen
Procédé pour la préparation de liposomes

(30) Priority: 14.06.1990 JP 153977/90; 24.05.1991 JP 148195/91
(43) Date of publication of application: 18.12.1991
(73) Proprietor: CANON KABUSHIKI KAISHA, Tokyo (JP)
(72) Inventor: Tomida, Yasuko, Canon Kabushiki Kaisha, Ohta-ku, Tokyo (JP); Ohyama, Junji, Canon Kabushiki Kaisha, Ohta-ku, Tokyo (JP); Sakuranaga, Masanori, Canon Kabushiki Kaisha, Ohta-ku, Tokyo (JP)
(74) Representative: Bühling, Gerhard, Dipl.-Chem.

(56) References cited:
- EP-A- 0 355 847
- ANGEWANDTE CHEMIE, vol. 27, no. 1, January 1988, pages 113-158; H. RINGSDORF et al.: "Molecular architecture and function of polymeric oriented systems: Models for the study of organization, surface recognition, and dynamics of biomembranes"
- J. BIOCHEM., vol. 101, no. 2, February 1987, pages 433-440; K. HIGASHI et al.: "Preparation and some properties of giant liposomes and proteoliposomes"
- BIOCHEMISTRY, vol. 27, no. 21, May 1988, pages 8261-8269, American Chemical Society; D. NEEDHAM et al.: "Structure and mechanical properties of giant lipid(DMPC) vesicle bilayers from 20 degrees Celsius below to 10 degrees Celsius above the liquid crystal-crystalline phase transition at 24 degrees Celsius"
- BIOCHEMISTRY, vol. 29, no. 51, 25th December 1990, pages 11215-11222, American Chemical Society; G. RIQUELME et al.: "Giant liposomes: A model system in which to obtain patch-clamp recordings of ionic channels"
- Biochimica et Biophysica Acta, 803 (1984), 145 - 152

## Description

### BACKGROUND OF THE INVENTION

### Field of the Invention

This invention relates to a process for preparing liposome, more particularly to a simple process for preparing giant liposome.

### Related Background Art

Liposomes which a closed vesicle of lipid can include various substances in its inner cavity or membrane, and therefore a number of studies have been made as the in vivo carrier of Pharmaceuticals. Also, liposome is useful as the model of biological membrane, and liposome alone or as proteolipsome having various membrane proteins fixed thereon has been widely utilized for the studies of various biological physicochemical properties. Further, recently, it has been also applied to cosmetics and foods.

As the preparation method of liposome, various methods have been known.

For example, there have been known the vortex method in which lipid is dissolved in an organic solvent, the organic solvent is removed in a glass vessel such as a kjeldahl flask to form the lipid thin film on the inner surface of the vessel, and then vortex treatment is carried out with addition of an aqueous solution [K. Inoue; Biochim. Biophys. Acta., 339, 390 (1974)] , the sonication treatment method in which sonication treatment is effected with addition of an aqueous solution to lipid thin film [L. Saunders, J. Perrin, D.B. Gammack; J. Pharm. Pharmacol., 14, 567 (1962)] , the reverse phase evaporation method in which emulsion is prepared by addition of an aqueous solution into an organic solvent containing lipid dissolved therein [F. Szoka, F. Olson, T. Heath, W. Vail, E. Mayhew, D. Papahadjopoulos; Biochim. Biophys. Acta., 601, 559 (1980)] , the detergent removal method in which the detergent is removed from mixed micell of lipid and detergent by dialysis [L.T. Mimms, G. Zampighi, Y. Nozaki, C. Tanford, J.A. Reynolds; Biochemistry, 20, 833 (1981)] , the freeze-thaw method in which freeze-thaw is effected after the sonication treatment [U. Pick; Arch. Biochem. Biophys, 212, 186 (1981)] and the calcium fusion method in which after the sonication treatment, CaCl₂ is added and treatment with EDTA is carried out [D. Papahadjopoulos, W.J. Vail, K. Jacobson, G. Poste; Biochim. Biophys. Acta., 394, 483 (1975)], and according to these methods, multi lamellar liposome, small unilamellar liposome and large unilamellar liposome can be prepared.

As the preparation method of giant liposome, there may be included the stationary hydration method in which the lipid thin film is hydrated over a long time without any stirring or vortexing [A. Darszon, C.A. Vandenberg, M. Schoufeld, M.H. Ellismau, N.C. Spitzer, M. Montal; Proc. Natl. Acad. Sci.; U.S.A., 77, 239 (1980)] , the freeze-thaw dialysis method in which dialysis is carried out against a buffer after the freeze-thaw in the presence of high salt concentration [N. Oku, R.C. MacDonald; Biochemistry, 22, 855 (1983)] , and giant liposome of unilamellar or unilamellar to plural lamellar membrane can be obtained according to these methods.

H. Ringsdorf, B. Schlarb and J.Venzmer, Angewandte Chemie vol. 27, no. 1 (Jan. 1988), p. 135, describe a flow chamber in which giant liposomes are prepared directly by swelling a lipid film. By using a syringe, the the medium in the flow chamber can be exchanged or additional components can be injected.

According to a method described by D.Needham and E.Evans, Biochemistry vo. 27, no. 21 (May 1988), p. 8262, liposomes are prepared by drying lipid in a chloroform-methanol solution onto a substrate, which includes solvent evaporation and complete drying overnight, and then rehydrating the dried lipid film at a particular temperature to produce vesicles.

In the studies of substance transfer through membrane, formation of biological membrane model, development of functional film, usefulness of the technique for forming artificially liposome is becoming higher.

Particularly, giant liposome with a diameter over several micrometers has such advantages that it can ensure a large holding volume, that it can hold a polymeric substance, that observation, manipulation through an optical microscope are possible, and therefore its usefulness is extremely high.

Whereas, although small liposomes with a diameter of about some 10 nm can be prepared simply and within a short time by utilizing the sonication treatment method, preparation of giant liposome by use of the above-described methods will require several days, and these methods cannot necessarily be said to be satisfactory in practical aspect.

### SUMMARY OF THE INVENTION

An object of the present invention is to provide a process which can prepare giant liposomes simply and within a short time.

Accordingly, the present invention provides a process for preparing liposome comprising the steps of:
(a) providing a pair of parallel substrates arranged to form a gap of 0.03 to 5 mm between said substrates;
(b) filling said gap with a liquid dispersion of a lipid capable of forming a liposome;
(c) drying said liquid dispersion to form a dried film in said gap; and
(d) hydrating said dried film by conacting said dried film with a liquid medium in said gap.

### BRIEF DESCRIPTION OF THE DRAWINGS

Fig. 1, Fig. 2, Fig. 4 and Fig. 5 are illustrations showing the states of the substrates arranged for preparation of liposomes according to the process of the present invention, Fig. 1 and Fig. 4 showing plan views thereof, Fig. 2 and Fig. 5 sectional views.

Fig. 6 is an illustration showing a photograph of the particle structure of the giant liposomes taken through an optical microscope prepared in Example 1 of the present invention, and Fig. 3 a graph showing the size distribution by optical microscopic observation of the giant liposomes prepared in Example 1.

### DETAILED DESCRIPTION OF THE PREFERRED EMBODIMENTS

As the material for liposome formation to be used in the present invention, any material capable of forming liposome can be utilized. For example, compounds having amphipilic property known in the art can be utilized. specifically, there may be employed lipids comprising phospholipids, glycolipids, quarternary ammonium salts.

The lipid molecule having the film forming ability is constituted of a long chain alkyl group having 8 or more carbon atoms and a hydrophilic group, and the hydrophilic may be any of cations such as: 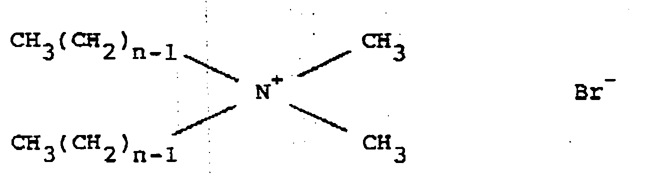 (n ≧ 4);
anions such as: 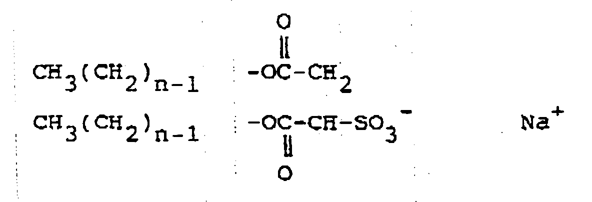 (n ≧ 4);
nonions such as: 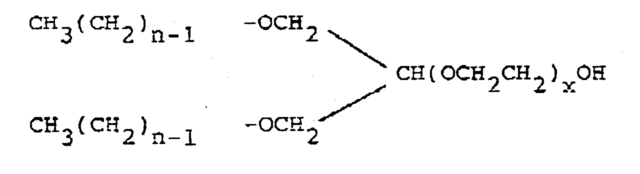 (n ≧ 4, x ≧ 1);
zwitterions such as: 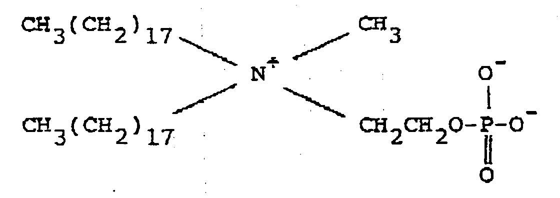 Among these lipid materials, glycerophospholipids such as phosphatidylcholine (lecithin), phosphatidyl ethanolamine, diphosphatidyl glycerol; sphingophospholipids such as sphingomyellin, ceramide ciliatine; sphingoglycolipids such as cerebroside, sulphatide, ceramide oligohexoside; and glyceroglycolipids such as glycosyl diacyl glycerol, containing carbohydrate as the hydrophilic group are preferable lipid materials, of which phospholipids such as glycerophospholipids, sphingophospholipids are particularly preferred.

The content of the above material for liposome formation in the liquid for liposome formation is not particularly limited, but it should be preferably 0.01 to 50 parts by weight, more preferably 0.1 to 20 parts by weight, based on 100 parts by weight of the liquid medium.

In the liquid for liposome formation, sterols such as cholesterol, cholestanol as the film stabilizer, charged substances such as stearylamine, phosphatidinic acid, dicetylphosphate, etc., antioxidants such as tocopherol may be also added.

The content of these additives may be preferably, for example, 0 to 2 parts by weight of sterols, 0 to 0.5 part by weight of charged substances and 0 to 0.2 part by weight of antioxidants based on 1 part by weight of the material for liposome formation.

As the liquid medium to be used for preparation of the liquid for liposome formation or the liquid medium to be added to the dried product, there may be included aqueous liquid media such as water; aqueous inorganic salt solution of potassium chloride, sodium chloride; aqueous sugar solutions of glucose, sucrose, dextrin. Also, various substances, for example, proteins such as enzymes, pharmaceuticals can be also contained in the aqueous liquid medium.

The content of the above inorganic salts and sugars in the aqueous liquid medium should be desirably made 0.01 to 30 parts by weight, preferably 0.1 to 15 parts by weight, based on 100 parts by weight of the aqueous liquid medium.

In the present invention, the liquid for liposome formation is inserted between the substrates opposed to each other with a predetermined interval.

The substrate is not limited, provided that the lamellar structure can be formed with the material for liposome formation can be formed or the liposomes can be gathered to form a mass, but those made of a glass are preferred with respect to the process of removing the liquid medium from the liquid by evaporation for liposome formation, namely wettability during concentration, drying and with repsect of easiness in availability.

The predetermined interval between the substrates can be easily set by, for example, providing a spacer. As such a spacer, for example, a silicone rubber can be utilized.

The size of the substrate may vary variously depending on the size, amount of the liposomes prepared. The interval between the substrates depends on the size of the desired liposome, and the interval between the innerside surfaces can be controled to the specified range of 0.03 to 5 mm, preferably 0.05 to 3 mm, more preferably 0.05 to 1 mm.

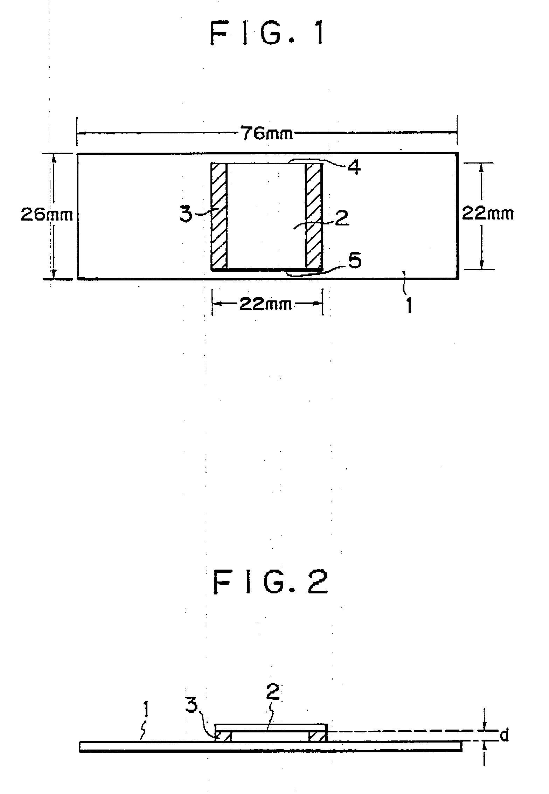

Fig. 1 and Fig. 2 show an example of the arrangement illustration having a pair of substrates for liposome formation arranged with a predetermined interval. In this example, as the upper substrate, a cover glass is employed, a slide glass as the lower substrate and a silicone rubber as the spacer, with the interval d between the upper and lower substrates being about 0.1 mm.

The liposome forming material can be used in any exsisting state in the liquid for liposome formation to be filled within the so called cavity between the substrates. For example, it can be used as small unilamellar liposome prepared by sonication treatment method, or alternatively the lipid thin film may be merely subjected to vortex treatment. Further, it is also possible to incorporate a substance such as protein in the liquid for liposome formation, or use a proteoliposome containing a protein, and in this case, giant proteoliposomes containing internally proteins can be formed within a short time.

Removal by evaporation of the liquid medium from the liquid for liposome formation containing the liposome forming material filled between the substrates, namely concentration and drying, can be performed at room temperature, but when it is desired to perpare liposomes within a short time, for example, heatig by an oven may be also utilized. The heating temperature in that case may be conveniently chosen depending on the composition of the liquid for liposome formation. It is not required that both sides represented by 4 and 5 in the arrangement illustration of the substrate shown in Fig. 1 should be vacant, provided that the liquid medium can be evaporated, but one of them may be clogged.

Hydration after concentration and drying can be executed by inserting the liquid medium (particularly water) between the substrates and leaving it to stand stationarily, but when the phase transition temperature of the material for liposome formation is room temperature or higher, hydration is effected while heating to the inherent temperature of the lipid material or higher.

The mechanism for formation of giant liposomes within a short time according to the process of the present invention may be estimated to be as follows. That is, since liposomes can be easily prepared when the material for liposome formation is hydrated to be under the state forming a lamellar structure (bilayer film), when once hydrated to form a lamellar structure or liposome and then dried under appropriate conditions, followed again by rehydration, giant liposome may be considered to be formed by a simple step. Here, if drying or hydration is performed from the layer of the liquid formed with a predetermined interval, the lamella phase or liposome will be gathered along the substrate surface with evaporation or hydration of the liquid medium to be concentrated, and fusion, swelling of these will readily occur during the step of hydration again after drying, whereby giant liposomes may be estimated to be formed within a short time.

Shortly speaking, in the present invention, for the purpose of making lamellar phase of liposomes readily gatherable along the substrate surface, a liquid containing the material for liposome formation and a liquid medium, there is employed the step of inserting the liquid so that the film thickness of the layer of said liquid may be formed at a predetermined interval, or the step of adding water to the dried product having the material for liposome formation so that the film thickness of said water layer may be formed at a predetermined interval.

The dried product obtained in the above step is arranged so as to fill the predetermined interval.

By preparing liposomes according to the present invention as described above, giant liposomes can be prepared simply within a short time, and can be applied as the model substance for biological membrane research, or the carrier for pharmaceuticals.

The present invention is described in more detail by referring to Examples.

### Example 1

An amount 0.5 ml of 20 mM Azolectin (soybean phosphatidylcholine, type IV S; manufactured by Sigma)-chloroform solution was poured into an kjeldahl flask, and after evaporation of the solvent by use of a rotary evaporator, placed in a dessicator and the solvent was completely removed by use of a vacuum pump to prepare a lipid thin film. Subsequently, 0.5 ml of 0.1 M aqueous potassium chloride was added, and vortexed for 2 minutes to have the lipid tin film dispersed in the solution.

20 µl aliguot of the above suspension of lipid thin film was injected and filled between the glass substrates shown in Fig. 1, and left to stand under an atmosphere of 20 °C, 30 % RH for 4 hours, to concentrate and dry. Then, 20 µl of water was injected and filled, and further left to stand stationarily 30 for minutes to prepare giant liposomes.

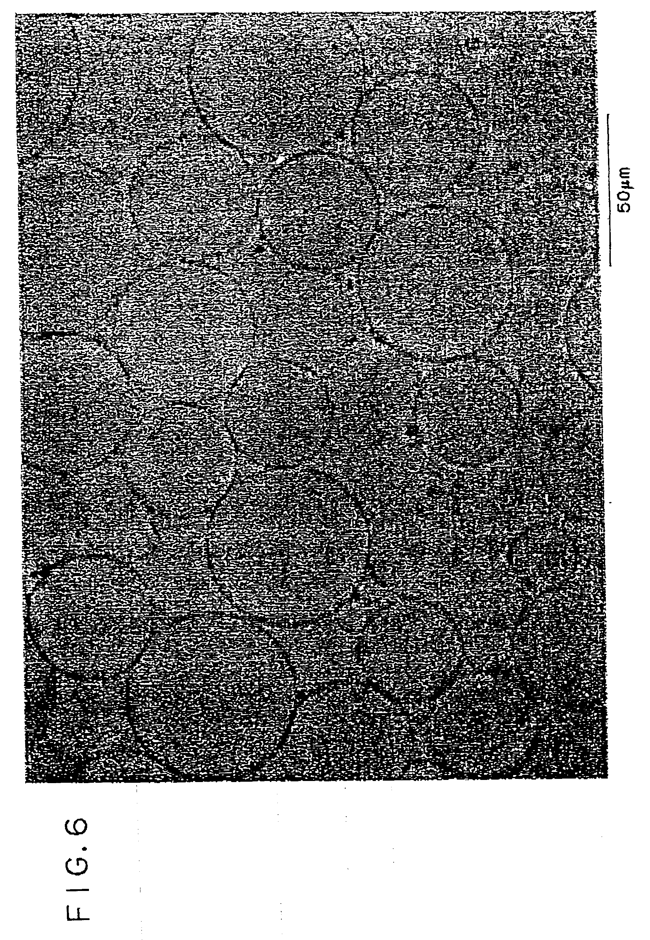

Fig. 6 shows the optical microscopic feature of the giant liposomes obtained. As seen in Fig. 6, a large nubmer of giant liposomes with diameters of some 10 µm were found to be formed.

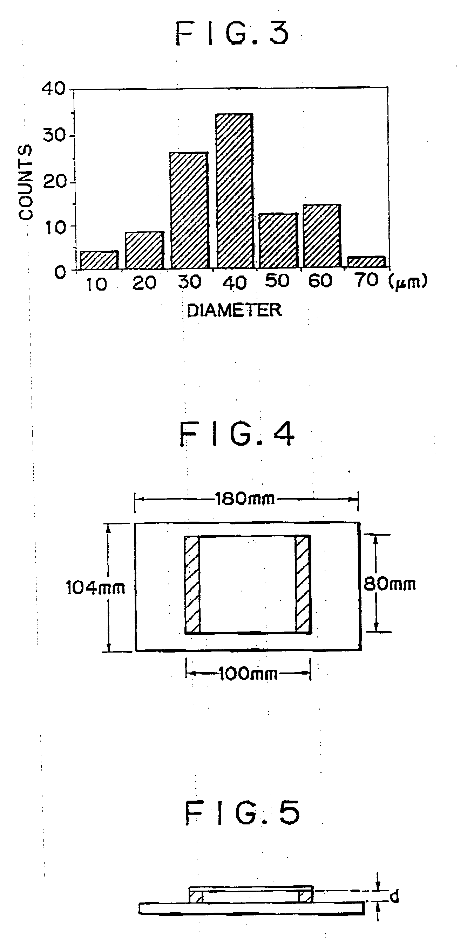

Next, Fig. 3 shows the size distribution of the giant liposomes by optical microscopic observation. The average particle size of the giant liposomes was 36 µm, and the standard deviation 13 (sample number 50).

### Example 2

After the lipid thin film was formed in the same manner as in Example 1, 0.5 ml of 0.02 M aqueous sodium chloride solution was added and vortexed for one minute, and sonicated in a bath-type apparatus (Sonifier B-15 Model, hop horn used; manufactured by Branson) for 30 minutes to obtain liposome dispersion.

The above liposome dispersion (20 µl) was injected and filled between the substrates similarly as in Example 1, and after concentration and drying, hydrated to prepare giant liposomes with an average particle size of 32 µm.

### Example 3

After the lipid thin film was formed in the same manner as in Example 1, 0.5 ml of 0.1 M aqueous glucose solution was added, and vortexed for 2 minutes to have the lipid thin film dispersed in the solution.

Next, 0.5 ml of the lipid thin film suspension was injected and filled between the glass substrates shown in Fig. 4 and Fig. 5 (interval d was about 0.1 mm), concentrated and dried in an oven of 50 °C for 2 hours. Then, 0.5 ml of water was injected and filled, followed further by leaving to stand stationarily for one hour to prepare giant liposomes with an average particle size of 48 µm.

### Example 4

A solution prepared by adding 13.5 mg of egg yolk phosphatidylcholine (type III-E; manufactured by Sigma) and 1.5 mg of stearylamine to chloroform (1 ml) was poured into an eggplant type flask, and after the lipid thin film was formed in the same manner as in Example 1, 1 ml of 0.05 M aqueous saccharose solution was added, and vortexed for 3 minutes to have the lipid thin film dispersed in the solution.

And, 0.7 ml of the lipid thin film suspension was concentrated and dried between the substrates similarly as in Example 3, and then 1 ml of water was added, followed by leaving to stand stationarily for one hour to prepare giant liposomes with an average particle size of 36 µm.

### Example 5

The purple membrane extracted from Halobacterium halobium R1 stain according to the method of Oesterhelt et al was treated with a detergent Triton X-100 (manufactured by Wako Junyaku) followed the method of Huang et al to remove the lipid from the purple membrane and obtain bacteriorhodopsin which is a membrane protein.

The above bacteriorhodopsin in an amount of 60 µg was added to 0.5 ml of the liposome suspension obtained in the same manner as in Example 2, and treated with a water bath type sonication apparatus (Sonifier B-15 Model, hop horn used; manufactured by Brauson) for 3 minutes to obtain a proteoliposome dispersion.

The proteoliposome suspension obtained was concentrated, dried between the substrates similarly as in Example 1, and then hydrated to prepare giant proteoliposomes with an average particle size of 36 µm.

According to the liposome preparation process of the present invention, by injecting, filling an aqueous liquid medium containing a liposome forming material between the substrates, and hydrating after drying, it has become possible to prepare giant liposomes with diameters exceeding some 10 µm simply, and within short time as compared with the prior art.

Since the giant liposomes have internally large holding volume, they can be utilized as the carrier for holding pharmaceuticals or proteins such as enzyme, and also since observation through an optical microscope can be practiced, they can be effectively utilized for basic research and application of biochemistry.

## Claims

1. A process for preparing liposomes comprising the steps of:
(a) providing a pair of parallel substrates arranged to form a gap of 0.03 to 5 mm between said substrates;
(b) filling said gap with a liquid dispersion of a lipid capable of forming a liposome;
(c) drying said liquid dispersion to form a dried film in said gap; and
(d) hydrating said dried film by contacting said dried film with a liquid medium in said gap.

2. A process for preparing liposomes according to Claim 1, wherein said material for liposome formation is a phospholipid.

3. A process for preparing liposomes according to Claim 1, wherein said liquid medium contains salt.

4. A process for preparing liposomes according to Claim 1, wherein said liquid medium contains sugar.

5. A process for preparing liposomes according to Claim 1, wherein said substrate is a glass.

6. A process for preparing liposomes according to Claim 1, wherein said liquid medium contains proteins.

7. A process for preparing liposomes according to any of the claims 1 to 6 comprising:
the step of adding a liquid medium to the residue obtained by said drying so that the film thickness of the layer of said liquid medium may be formed at said predetermined interval.

## Patentansprüche

1. Verfahren zum Herstellen von Liposomen, umfassend die Schritte:
(a) Bereitstellen eines Paars paralleler Substrate, die so angeordnet sind, daß sich zwischen ihnen ein Spalt von 0,03 bis 5 mm bildet;
(b) Auffüllen des Spalts mit einer flüssigen Dispersion eines Lipids, das in der Lage ist, Liposomen zu bilden;
(c) Trocknen der flüssigen Dispersion, um einen getrockneten Film in dem Spalt zu bilden; und
(d) Hydratisieren des getrockneten Films, indem der getrocknete Film in Kontakt mit einem flüssigen Medium in dem Spalt gebracht wird.

2. Verfahren zum Herstellen von Liposomen nach Anspruch 1, wobei es sich bei dem Material zur Bildung von Liposomen um Phospholipid handelt.

3. Verfahren zum Herstellen von Liposomen nach Anspruch 1, wobei das flüssige Medium Salz enthält.

4. Verfahren zum Herstellen von Liposomen nach Anspruch 1, wobei das flüssige Medium Zucker enthält.

5. Verfahren zum Herstellen von Liposomen nach Anspruch 1, wobei das Substrat Glas ist.

6. Verfahren zum Herstellen von Liposomen nach Anspruch 1, wobei das flüssige Medium Proteine enthält.

7. Verfahren zum Herstellen von Liposomen nach einem der Ansprüche 1 bis 6, umfassend:
den Schritt der Zugabe eines flüssigen Mediums zu dem Rest, welcher durch Trocknenlassen erhalten wird, so daß die Filmdicke der Schicht von flüssigem Medium innerhalb des vorher bestimmten Intervalls geformt werden kann.

## Revendications

1. Procédé de production de liposomes, qui comprend les étapes consistant :
(a) à prendre une paire de substrats parallèles agencés de manière à former entre eux un espace de 0,03 à 5 mm ;
(b) à garnir cet espace d'une dispersion liquide d'un lipide capable de former un liposome ;
(c) à sécher cette dispersion liquide pour former un film séché dans l'espace en question ; et
(d) à hydrater ce film séché par sa mise en contact avec un milieu liquide présent dans l'espace en question.

2. Procédé de production de liposomes suivant la revendication 1, dans lequel la matière utilisée pour la formation des liposomes est un phospholipide.

3. Procédé de production de liposomes suivant la revendication 1, dans lequel le milieu liquide en question contient un sel.

4. Procédé de production de liposomes suivant la revendication 1, dans lequel le milieu liquide en question contient un sucre.

5. Procédé de production de liposomes suivant la revendication 1, dans lequel le substrat en question est un verre.

6. Procédé de production de liposomes suivant la revendication 1, dans lequel le milieu liquide en question contient des protéines.

7. Procédé de production de liposomes suivant l'une quelconque des revendications 1 à 6, comprenant :
l'étape d'addition d'un milieu liquide au résidu obtenu par l'opération de séchage en question de manière que l'épaisseur de film de la couche de ce milieu liquide puisse être formée dans l'intervalle prédéterminé mentionné.
